(19) Europäisches Patentamt
European Patent Office
Office européen des brevets

(11) Publication number: **0 295 604**
**A1**

(12) **EUROPEAN PATENT APPLICATION**

(21) Application number: **88109375.1**

(22) Date of filing: **13.06.88**

(51) Int. Cl.⁴: **C07C 87/29 , A61K 31/135 , //(A61K31/135,31:195)**

Claims for the following Contracting States: ES + GR.

(30) Priority: **16.06.87 US 62758**

(43) Date of publication of application: **21.12.88 Bulletin 88/51**

(84) Designated Contracting States: **AT BE CH DE ES FR GB GR IT LI LU NL SE**

(71) Applicant: **MERRELL DOW PHARMACEUTICALS INC.**
**2110 East Galbraith Road**
**Cincinnati Ohio 45215-6300(US)**

(72) Inventor: **McDonald, Ian A.**
**9382 Kentonsrun Court**
**Loveland Ohio 45140(US)**
Inventor: **Palfreyman, Michael G.**
**11515 Applejack Court**
**Cincinnati Ohio 45249(US)**

(74) Representative: **Macchetta, Francesco et al**
**Gruppo Lepetit S.p.A. Patent and Trademark Department 34, Via Roberto Lepetit**
**I-21040 Gerenzano (Varese)(IT)**

(54) **(E)-2-(p-Fluorophenethyl)-3-fluoroallylamine.**

(57) This invention related to (E)-2-(p-fluorophenethyl)-3-fluoroallylamine, to its method of preparation and to its pharmacological activity inhibiting monoamine oxidase-B and to its end-use application in the treatment of Parkinson's Disease.

EP 0 295 604 A1

## (E)-2-(p-FLUOROPHENETHYL)-3-FLUOROALLYLAMINE

This invention related to (E)-2-(p-fluorophenethyl)-3-fluoroallylamine, to its method of preparation and to its pharmacological activity inhibiting monoamine oxidase-B and to its end-use application in the treatment of Parkinson's Disease.

More specifically, this invention relates to 2-(p-fluorophenethyl)-3-fluoroallylamine, a compound having the structural formula

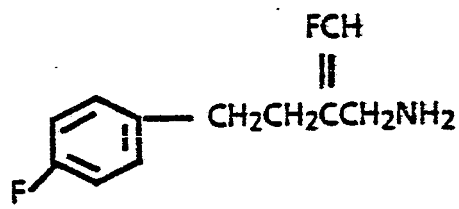

and the pharmaceutically acceptable salts thereof, a potent and selective enzyme-activated, irreversible inhibitor of MAO-B, useful in the treatment of patients suffering from Parkinson's Disease.

Appropriate pharmaceutically acceptable and addition salts are such salts derived from organic and inorganic acids such as hydrochloric, hydrobromic, sulfonic, sulfuric, phosphoric, nitric, maleic, fumaric, benzoic, ascorbic, pamoic, succinic, methanesulfonic, acetic, propionic, tartaric, citric, lactic, malic, mandelic, cinnamic, palmitic, itaconic, and benzenesulfonic.

In general, the preparation of the compound of this invention may be effected by procedures well known in the art, (e.g., U.S. Patent 4,454,158) wherein a diester of p-fluorophenylethylbutyric acid is halomethylated in known manner by first treating the diester with a strong base to produce the corresponding carbanion and then contacting the carbanion with a suitable halomethylating agent. The strong base must be non-nucleophilic and be of sufficient strength to remove a proton from the methine moiety adjacent to the carboxy group of the starting ester. Suitable such bases are known in the art. Examples are: (a) an alkyl lithium (e.g., n-butyllithium), (b) an aryl lithium (e.g., phenyllithium), (c) a lithium dialkylamide (e.g., lithium diisopropylamide), (d) sodium or lithium amide, (e) a metal hydride (e.g., sodium or potassium hydride), (f) metal alcoholate (e.g., sodium or potassium tert-butoxide), or (g) lithium or dilithium acetylide. The reaction between the diester and the base can be performed in an aprotic organic solvent (such as tetrahydrofuran (THF), diethyl ether, dimethylformamide (DMF), dimethyl sulfoxide (DMSO), dimethoxyethane, or dioxane, or mixtures thereof), using a temperature range of about 0° to 70° C, preferably 45° C, and a reaction time of about 5 minutes to 2 hours. Preferred bases for forming the carbanion are sodium hydride in dimethoxyethane, potassium tert-butoxide/n-butyllithium in THF, or sodium tert-butoxide in THF.

Following halomethylation it is preferred to selectively remove one of the ester groups by acid hydrolysis. To accomplish selective cleavage it is preferred to have a mixed diester wherein one ester group is easily cleaved (e.g., one ester group bears t-butyl, benzyl, diphenylmethyl or triphenylmethyl) while the other bears a straight chain alkyl (e.g., methyl, ethyl, propyl or n-butyl.

The easily cleaved ester group can be selectively hydrolyzed by treatment with an organic or inorganic acid, either with or without an added solvent, using a temperature range of about 0° to 25° C and a reaction time of about 1 to 10 hours. Ambient temperature is preferred. The choice of the acid for the hydrolysis is not critical, except that the acid should be chosen so that it can be easily removed after the hydrolysis stage. Trifluoroacetic acid is preferred since its low boiling point permits it to be easily removed from the hydrolysis product. When one ester group bears benzyl, diphenylmethyl, or triphenylmethyl and the other is a straight-chain $C_1$-$C_4$ alkyl group, the easily cleaved ester group can also be selectively cleaved by subjecting the mixed diester to catalytic hydrogenolysis using conventional procedures: for example, by treatment under a hydrogen atmosphere in the presence of a catalyst (e.g., Pd/C) at ambient temperature for 1 to 48 hours. As will be apparent to those skilled in the art, the ester groups can be chosen so that both groups can be cleaved simultaneously by acid hydrolysis or catalytic hydrogenolysis.

Following selective hydrolysis, the halomethylated monoester is converted to its acrylate ester by treatment with a base. The reaction can be performed using an aqueous or non-aqueous solvent with strong bases such as sodium hydroxide and the like or weak bases, such as triethylamine or sodium bicarbonate, can be used. With strong bases, care must be exercised to avoid using an excess of base to avoid interaction with the double bond. The choice of the base, the reaction solvent and reaction conditions will be

apparent to those skilled in the art. A preferred procedure is to use aqueous sodium hydroxide in THF at ambient temperature. In general, a temperature range of 0° to 25° C and reaction time of 15 minutes to 2 hours can be used.

The acrylate ester is reduced to yield the allyl alcohol. The reducing agent employed for this transformation can be any reagent which is known in the art to be capable of selectively reducing an ester function of carboxylic acid function to the corresponding carbinol in the presence of a double bond. A preferred reducing agent is diisobutylaluminum hydride (DIBAL-H) in hexane, THF, diethyl ether, or dichloromethane, or mixtures thereof. In a preferred procedure, a solution of the acrylate methyl ester in THF is cooled to about 0° to -78° C (preferably -60° to -70° C), the DIBAL-H dissolved in hexane is added, and the temperature of the mixture is allowed to rise to ambience. The reaction time can be about 2 to 24 hours.

The allyl alcohol can be converted to the desired allyl primary amine using procedures known in the art to be useful for replacing an allylic hydroxyl group by an allylic primary amino group. A preferred laboratory method involves the direct formation of an imido derivative, preferably the phthalimide, and subsequent cleavage of the imido group to generate the primary amino group. The imido derivative can be prepared conveniently by treating the allyl alcohol with the appropriate imide (i.e., phthalimide, succinimide, or maleimide) in the presence of a triarylphosphine (e.g., triphenylphosphine) or a trialkylphosphine and diethyl azodicarboxylate in an aprotic organic solvent (e.g., THF or dioxane). The reaction can be performed using a temperature range of about 0° to 70° C and a reaction time of about 1 to 24 hours. Ambient temperature is preferred. The imido derivative can be cleaved, preferably by reaction with hydrazine in an organic solvent, such as an alkanol (e.g., ethanol) at reflux temperature (50° to 100° C) and a reaction time of about 30 minutes to 10 hours. It is preferably to add an acid (e.g., hydrochloric acid) after the hydrazine treatment to convert the product to the acid addition salt. Other reagents can be used to cleave the imido function. For example, the imide can be heated with a strong mineral acid (e.g., hydrochloric or sulfuric acid) or a mixture of hydrochloric acid and acetic acid. Acids, such as hydrobromic acid, which are reactive towards olefins usually cannot be used. The final products are conveniently purified and isolated as the acid addition salt using conventional purification methods.

The foregoing procedures may be illustrated by the following examples.

## EXAMPLE 1

### 2-(p-Fluorophenethyl)-3-fluoroallylamine HC1

**Step A.** Ethyl 2-(tert-butoxycarbonyl)-p-fluorophenylbutyrate

A solution of p-fluorophenylbutyric acid (25 g) in tert-butyl acetate (349 ml) is treated with perchloric acid (1.77 ml) then stirred at ambient temperature for 1.5 hours. This is subsequently poured into water (350 ml) containing NaOH (48 g) and the tert-butyl ester is isolated by ether extraction as a pale yellow oil. A solution of lithium diisopropylamide is prepared from diisopropylamine (22.74 g) and 1.6M n-butyl lithium (143.7 ml) in THF (200 ml). This is cooled to -78° C and a solution of tert-butyl p-fluorophenylbutyrate (26.76 g) in THF (100 ml) is added slowly. After 1 hour a solution of ethyl chloroformate (12.19 g) in THF (100 ml) is added and stirring is continued at ambient temperature for 24 hours. The mixture is then poured into water, neutralized with dilute aqueous HCl and the product isolated by ether extraction. In this way the crude malonate is obtained as an orange oil (32.27 g).

**Step B.** Ethyl 2-(tert-butoxycarbonyl)-2-(difluoromethyl-p-fluorophenylbutyrate

Solid sodium tert-butoxide (19.81 g) is added to a solution of crude ethyl 2-(tert-butoxycarbonyl)-p-fluorophenylbutyrate (32.14 g) in THF (400 ml). The mixture is stirred for 1 hour then heated to 45° C at which time $ClCHF_2$ gas is added rapidly for about 15 minutes. Stirring is continued for 1 hour under an atmosphere of $ClCHF_2$ during which time the temperature falls to ambient. The reaction mixture is poured into water/brine and the crude product is isolated as an orange oil (34.55 g) by ether extraction.

**Step C.** (E)-Ethyl 2-(p-fluorophenethyl)-3-fluoroacrylate

A solution of ethyl 2-(tert-butoxycarbonyl)-2-(difluoromethyl)-p-fluorophenylbutyrate (30.28 g) in trifluoroacetic acid (168 ml) is stirred for 1 hour, then the excess trifluoroacetic acid is removed by evaporation. The residual oil (25.82 g) is dissolved in THF (230 ml) and treated slowly with 2M NaOH (80 ml) so that the pH does not rise above 7.02. After completion of the addition the solution is stirred for another 15 minutes and the product is extracted into ether. The ether is evaporated and the residue is filtered through a short column of silica using 5% EtoAc in light petroleum as solvent. Evaporation of the solvent affords essentially pure product as a pale orange oil (15.75 g).

**Step D.** (E)-2-(p-Fluorophenethyl)-3-fluoroallyl alcohol

A solution of the acrylate (15.70 g) in hexane (350 ml) cooled to -10° is treated slowly with a solution of diisobutylaluminum hydride in hexane (1M solution, 196 ml). THe solution is stirred at ambient temperature for 90 minutes, then cooled to 10° C and treated consecutively with $CH_3OH$ (196 ml) and 6M aqueous HCl (245 ml) . Water is added and the product is isolated by ether extraction followed by distillation of the solvents to leave almost pure alcohol (11.36 g).

**Step E.** (E)-1-Fluoro-2-(p-fluorophenethyl)-3-phthalimidopropene

A solution of the crude alcohol (11.36 g) phthalimide (8.43 g) and triphenylphosphine (5.03 g) in THF (400 ml) is cooled to 0° C and treated slowly with a solution of diethyl azodicarboxylate (9.99 g) in THF (50 ml). Stirring is continued at ambient temperature overnight, then the solution is evaporated to leave an orange paste (30 g). Chromatography on silica (20% EtoAc in petroleum ether as eluant) allows the separation of pure product (13.90 g) as a pale yellow solid).

**Step F.**

A mixture of the phthalimide (0.26 g) and hydrazine hydrate (80 mg) in ethanol (5 ml) is refluxed for 2.5 hours. 6N aqueous HCl (1.2 ml) is added and the mixture is evaporated to dryness. The residue is dissolved in NaOH (10 ml) and the crude amine is isolated by ether extraction then dissolved in THF (10 ml) and treated with di-tert-butyl dicarbonate (194 mg). The solution is refluxed for 2 hours then the crude N-BOC derivative is isolated by ether extraction. Purification is achieved by silica chromatography (25% EtoAc in petroleum ether) whereupon pure material (180 mg) is obtained as an almost colorless oil. This is dissolved in hydrogen chloride-saturated ether (12 ml), left overnight, then filtered to give the hydrochloride salt of (E)-2-(p-fluorophenethyl)-3-fluoroallylamine (30 mg) as colorless plates; m.p. 131° C.

Monoamine oxidase (MAO) inhibitors have been used clinically for over twenty years. In the 1970's it was realized that MAO was two enzymes with different distri butions, substrate and inhibitor sensitivities. In man, the Type A enzyme metabolizes primarily norepinephrine and serotonin, whereas the Type B enzyme is important for the metabolism of dopamine. MAO Type A is selectively inhibited by clorgyline. L-Deprenyl selectively inhibits MAO Type B. L-Deprenyl, in a large number of clinical trials, has been shown to be of benefit to Parkinsonian patients. The inhibitor is used in combination with L-Dopa and a decarboxylase inhibitor and has particular value in controlling the "on-off" effect and "end of dose" dyskinesias. Moreover, analysis of data from a large number of Parkinsonian patients treated with L-deprenyl plus Madopar (L-Dopa + benserazide) compared with Madopar alone suggests that L-deprenyl treatment significantly prolongs life expectancy. Relevant to this observation, it has been shown in mice and monkeys that L-deprenyl treatment

blocks the neurotoxic effect of MPTP (N-methyl-4-phenyl-1,2,3,6-tetrahydropyridine) which produces the Parkinsonian syndrome in man. Indeed, it has been suggested that an MPTP-like neurotoxin might be the cause of idioipathic Parkinson's disease.

In man and animals, L-deprenyl is rapidly transformed to L-amphetamine and methamphetamine, and these products may contribute to some of the side effects observed with L-deprenyl. Furthermore, L-deprenyl has only limited selectivity for the MAO-B enzyme. Thus a compound with enhanced selectivity for the MAO-B enzyme and devoid of amphetamine-like effects should of considerable benefit in Parkinson's disease. The compound of this invention has a biochemical and pharmacological profile that meets these criteria.

(E)-2-p-Fluorophenethyl)-3-fluoroallylamine is a potent and selective enzyme-activated, irreversible inhibitor of MAO-B. In vitro, the IC50 values for inhibition of rat brain mitochondrial MAO-A and -B are 6.8 x $10^{-7}$M and 3.6 x $10^{-9}$M, respectively; a selectivity ratio of 190 in favor of MAO-B inhibition. In vivo, the compound is active orally, is potent and with a selectivity ratio of 44 remains selective for MAO-B.

Following repeated daily administration for 14 days, (E)-2-p-fluorophenethyl)-3-fluoroallylamine remained selective for the MAO-B enzyme. From the irreversible inhibition, mode of action and the long half-life of the enzyme, a lower daily dose is needed to inhibit the enzyme. In the rat, the oral doses necessary to inhibit by 50% the MAO-A and MAO-B enzyme in various tissues are shown in Table 1.

**TABLE 1**

**Effect of chronic (14 day) oral administration of (E)-2-(p-fluorophenethyl)-3-fluoroallylamine on rat MAO activity**

| Tissue | ED50 mg/kg p.o. | |
|---|---|---|
| | MAO-A | MAO-B |
| Brain | > 5 | 0.025 |
| Heart | 5 | 0.5 |
| Liver | > 5 | 0.01 |
| Duodenum | >>5 | 0.25 |

MPTP given parenterally to mice depletes striatal dopamine. This effect can be prevented by MAO-B, but not by MAO-A inhibitor. (E)-2-p-Fluorophenethyl)-3-fluoroallylamine protects mice from the neurotoxic effect of MPTP as seen from Table 2.

| TABLE 2<br>Prevention of MPTP neurotoxicity | |
|---|---|
| Treatment | Striatal dopamine<br>ug/g ± SEM |
| Control | 15.2 ± 0.8 |
| MPTP (20 mg/kg i.p.) | 4.3 ± 0.4 |
| (E)-2-p-fluorophenethyl)-3-fluoroallylamine (1.25 mg/kg i.p.) | 16.6 ± 0.9 |
| MPTP plus (E)-2-p-fluorophenethyl)-3-fluoroallylamine | 16.0 ± 0.4 |

Non-selective and MAO-A selective inhibitors are known to produce dangerous cardiovascular interactions with tyramine containing foods; the so-called "cheese reaction". L-Deprenyl is devoid of this effect. Given acutely at 10 and 50 times the ED50 dose for inhibition of MAO-B, (E)-2-p-fluorophenethyl)-3-fluoroallylamine is also devoid of interactions with tyramine as seen in Table 3.

| Table 3<br>Potentiation of the cardiovascular effects of tyramine by MAO inhibitors administered orally | | |
|---|---|---|
| Treatment | Tyramine potentiation ratio* | |
| | i.d. | i.v. |
| Control | 1 | 1 |
| (E)-2-p-fluorophenethyl)-3-fluoroallylamine (1.8 mg/kg p.o.) | 1.4 | 0.8 |
| (E)-2-p-fluorophenethyl)-3-fluoroallylamine (9 mg/kg p.o.) | 1.3 | 1.8 |

*Tyramine potentiation was calculated from a dose response curve to the tachycardia response to intraduodenal (i.d.) tyramine (0.3-50 mg/kg) and intravenous (i.v.) tyramine (1.25-80 µg/kg) by comparing the ED50 dose of tyramine in control animals vs that in treated animals.

Thus in its end-use application for the treatment of Parkinson's Disease, (E)-2-p-fluorophenethyl)-3-fluoroallylamine may be used at dose ranges of about 0.01 to 1 mg per kilogram of body weight per day.

Of course, when employed to treat Parkinson's Disease the effective dosage of the compound of this invention will vary according to the nature and severity of the disease and the particular subject being treated. In general, effective results can be achieved by administering a compound at a dosage level of

from about 0.1 to 1 mg per day per kilogram of body weight. In practice, therapy should be initiated systemically at lower doses, the dosage thereafter being administered orally in solid dosage forms, e.g., capsules, tablets, or powders, or in liquid forms, e.g., solutions or suspensions. The compounds may also be injected parenterally in the form of sterile solutions or suspensions. Solid oral forms may contain conventional excipients, for instance, lactose, sucrose, magnesium stearate, resins, and like materials. Liquid oral forms may contain various flavoring, coloring, preserving, stabilizing, solubilizing, or suspending agents. If desired, additives, such as saline or glucose may be added to make the solutions isotonic. When the compounds of this invention are used in conjunctive therapy with L-DOPA lower amounts of L-DOPA are required and thus any side effects coherent with large amounts of L-DOPA will be lessened or obviated.

The term "conjunctive therapy" is intended to comprise simultaneous, separate or sequential administration.

**Claims**

1. (E)-2-(p-Fluorophenethyl)-3-fluoroallylamine or a pharmaceutically acceptable salt thereof.
2. (E)-2-(p-Fluorophenethyl)-3-fluoroallylamine.
3. A compound of claim 1 or 2 for use as a medicine.
4. Use of a compound of claim 1 or 2 for preparing a medicament for inhibiting monoamino-oxidase-B or treating Parkinson's disease.

A process for preparing a compound of claim 1 or 2 which comprises replacing the allylic hydroxy group of (E)-2-(p-fluorophenethyl)-3-fluoroallyl alcohol with a primary amino group to yield (E)-2-(p-fluorophenethyl)-3-fluoroallylamine.

6. A product comprising a compound of claim 1 or 2 and L-Dopa as a combined preparation for simultaneous, separate or sequential use in the treatment of Parkinson's disease.
7. A product comprising a compound of claim 1 or 2 and a peripheral inhibitor of aromatic L-aminoacid decarboxylase as a combined preparation for simultaneous, separate or sequential use in the treatment of Parkinson's disease.
8. A product comprising a compound of claim 1 or 2, L-Dopa and a peripheral inhibitor of aromatic L-aminoacid decarboxylase as a combined preparation for simultaneous, separate or sequential use in the treatment of Parkinson's disease.

Claims for the following contracting States: ES, GR

1. A process for preparing (E)-2-(p-fluorophenethyl)-3-fluoroallylamine or a pharmaceutically acceptable salt thereof which comprises replacing the allylic hydroxy group of (E)-2-(p-fluorophenethyl)-3-fluoroallyl alcohol with a primary amino group to yield (E)-2-(p-fluorophenethyl)-3-fluoroallylamine.
2. A process according to claim 1 which comprises:
   (a) reducing (E)-2-(p-fluorophenethyl)-3-fluoroaacrylic acid ester to yield (E)-2-(p-fluorophenethyl)-3-fluoroallyl alcohol, and
   (b) replacing the allylic hydroxy group of (E)-2-(p-fluorophenethyl)-3-fluoroallyl alcohol with a primary amino group to yield (E)-2-(p-fluorophenethyl)-3-fluoroallylamine.
3. A process according to claim 1 or 2 for preparing (E)-2-(p-fluorophenethyl)-3-fluoroallylamine.
4. Use of a compound of claim 1 or 2 for preparing a medicament for inhibiting monoamino-oxidase-B or treating Parkinson's disease.
5. A product comprising a compound of claim 1 or 2 and L-Dopa as a combined preparation for simultaneous, separate or sequential use in the treatment of Parkinson's disease.
6. A product comprising a compound of claim 1 or 2 and a peripheral inhibitor of aromatic L-aminoacid decarboxylase as a combined preparation for simultaneous, separate or sequential use in the treatment of Parkinson's disease.
7. A product comprising a compound of claim 1 or 2, L-Dopa and a peripheral inhibitor of aromatic L-aminoacid decarboxylase as a combined preparation for simultaneous, separate or sequential use in the treatment of Parkinson's disease.

European Patent Office

# EUROPEAN SEARCH REPORT

Application Number

EP 88 10 9375

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 224 924 (MERRELL DOW PHARMACEUTICALS) * Claims 1,9,13-19 * | 1,3-5 | C 07 C 87/29<br>A 61 K 31/135//<br>(A 61 K 31/135<br>A 61 K 31:195) |
| D,Y | EP-A-0 067 105 (NERRELL TORAUDE ET COMPAGNIE) * Claims * | 1,3-5 | |
| | | | **TECHNICAL FIELDS SEARCHED (Int. Cl.4)** |
| | | | C 07 C 87/00<br>A 61 K 31/00 |

The present search report has been drawn up for all claims

| Place of search | Date of completion of the search | Examiner |
|---|---|---|
| THE HAGUE | 21-09-1988 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)